# EUROPEAN PATENT SPECIFICATION

(11) **EP 3 443 951 B1**
(45) Date of publication and mention of the grant of the patent: **21.09.2022**
(21) Application number: 17782174.1
(22) Date of filing: 15.03.2017
(51) Int. Cl.: A61K 8/64, A61K 8/31, A61K 8/365, A61K 8/60, A61K 8/73, A61Q 1/14, A61Q 19/00, A61Q 19/10

(54) **SUPERFATTING AGENT AND PERSONAL CARE COMPOSITION**
ÜBERFETTUNGSMITTEL UND KÖRPERPFLEGEZUSAMMENSETZUNG
AGENT SURGRAISSANT ET COMPOSITION DE SOIN PERSONNEL

(30) Priority: 11.04.2016 JP 2016079083
(43) Date of publication of application: 20.02.2019
(73) Proprietor: Sabo S.p.A., 24040 Levate BG (IT); Kaneka Corporation, Osaka-shi, Osaka 530-8288 (JP)
(72) Inventor: ORI, Callot, 24040 Levate BG (IT); ENRICO, Costantini, 24040 Levate BG (IT); NAGANO, Takuto, Osaka-shi Osaka 530-8288 (JP); YANAGISAWA, Satohiro, Tokyo 107-6025 (JP)
(74) Representative: Hoffmann Eitle
(86) International application number: PCT/JP2017/010329
(87) International publication number: WO 2017/179356

(56) References cited:
- EP-A1- 0 995 487
- WO-A1-03/013446
- WO-A1-2015/133455
- FR-A1- 2 790 977
- FR-A1- 2 810 323
- JP-A- H0 925 223
- JP-A- 2000 327 591
- JP-A- 2001 316 222
- JP-A- 2002 145 761
- JP-A- 2003 176 211
- JP-A- 2003 267 836
- JP-A- 2005 097 284
- JP-A- 2006 265 153
- MAKOVITZKI ARIK ET AL: "Ultrashort antibacterial and antifungal lipopeptides", PROCEEDINGS OF THE NATIONAL ACADEMY OF SCIENCES OF THE UNITED STATES OF AMERICA, NATIONAL ACADEMY OF SCIENCES, US, vol. 103, no. 43, 1 October 2006 (2006-10-01), pages 15997-16002, XP009096818, ISSN: 0027-8424, DOI: 10.1073/PNAS.0606129103
- MNIF INÈS ET AL: "Lipopeptide surfactants: Production, recovery and pore forming capacity", PEPTIDES, vol. 71, 1 September 2015 (2015-09-01), pages 100-112, XP055580317, AMSTERDAM, NL ISSN: 0196-9781, DOI: 10.1016/j.peptides.2015.07.006

## Description

### TECHNICAL FIELD

The present invention relates to a super-fatting agent and a personal care formulation containing the super-fatting agent. More specifically, the invention relates to a super-fatting agent containing a lipopeptide compound as will be described herein, squalane and an an alkyl polyglycoside (APG), and a personal care formulation containing the super-fatting agent. The super-fatting agent according to the present invention is a really effective when incorporated into a personal care formulation, and exhibits the functions such as high skin softness, good distribution on the skin, cushion foam improvement, reduction of foam's bubbles size, rinse-off improvement, reduction of stickiness feeling on skin, very smoothness touch after application and long lasting.

### BACKGROUND ART

As a lipopeptide compound derived from a microorganism, for example, sodium surfactin is known as a raw material for cosmetics, which, for its low skin irritating property and favorable biodegradability, has attracted attention, and is disclosed in e.g. Patent Documents 1 to 3.

Squalane is a hydrocarbon and triterpene derived by hydrogenation of squalene, it is used as an extremely effective emollient and as a natural moisturizer in cosmetic applications with no toxicity, neither irritation, allergy nor sensitization and it' s not comedogenic, but it's very difficult to be dispersed by surfactants into a suitable gels for toiletries applications.

In particular, no cleansing cosmetic containing squalane in a good satisfying gel form or cream form having a good wash-ability has been provided till now. The present preparation dedicated to super-fatting toiletry formulations, comprising a lipopeptide compound derived from a microorganism, and an Alkyl polyglycoside (APG) in combination with squalane solve that issue.

Patent Document 4 describes emulsion compositions comprising 5-60 wt.% of a mixture of alkyl polyglycosides and 40-95 wt.% of one or more fatty alcohols.

Patent Docunment 5 describes water-in-oil emulsions containing: 5 to 70 wt.% of an aqueous phase; 20 to 90 wt.% of a fatty phase; and 3 to 25 wt.% of an emulsifier comprising: 10 to 90 wt.%, of a mixture in any proportion of an oleylglycoside having a degree of polymerisation ranging between 1 and 3 and of an isostearylglycoside having a degree of polymerisation ranging between 1 to 3; and 90 to 10 wt.% of a mixture in any proportion of oleic alcohol and isosterarylic alcohol.

Patent Document 6 describes the lipopeptide elaidyl-lysyl-phenylalanine-lysine(elaidyl-KFK) and cosmetic compositions containing it.

Non-patent document 1 describes a study in which it was found that (i) the attachment of an aliphatic chain to otherwise inert, cationic D,L tetrapeptides endows them with potent activity against various microorganisms including antibiotic resistance strains; (ii) cell specificity is determined by the sequence of the short peptidic chain and the length of the aliphatic moiety; and (iii) despite the fact that the peptidic chains are very short, their mode of action involves permeation and disintegration of membranes, similar to that of many long antimicrobial peptides.

### PRIOR ART DOCUMENT

### PATENT DOCUMENTS AND NON-PATENT DOCUMENTS

Patent Document 1: WO 99/62482
Patent Document 2: EP 1660025
Patent Document 3: JP 2003-176211 A
Patent Document 4: EP0995487 A2
Patent Document 5: FR2790977 A1
Patent Document 6: FR2810323 A1

Non-patent Document 1: Makovitzki et al., Proceedings of the National Academy of Sciences of the United States of America, National Academy of Sciences, 2006, vol. 103 (43), pp. 15997-15998.

### DISCLOSURE OF THE INVENTION

### PROBLEMS TO BE SOLVED BY THE INVENTION

An object of the present invention is to provide a personal care preparation which, being stable and having an extremely low skin irritating property, ensures high skin comfort, and exhibits a good wash-ability , good distribution on the skin, cushion foam improvement, reduction of foam's bubbles size, rinse-off improvement, reduction of stickiness feeling on skin, very smoothness touch after application and long lasting, when used in a cosmetic preparation or the like.

### MEANS FOR SOLVING THE PROBLEMS

As a result of intensive investigations to solve the above-described problem, the present inventors have found that the aforementioned object is attained by a super-fatting agent which comprises a lipopeptide compound as will be described herein, and an alkyl polyglycoside (APG) and squalane as a mixture representing a Lipid Layer Enhancer.

The super-fatting agent is then formulated together with others cosmetic ingredients like cleansing, emulsifying foaming agents, surfactants, antistatic, foam boosting, hair conditioning, skin conditioning, viscosity controlling agents, bulking, masking agents, buffering, humectant, skin conditioning agents, preservative, perfuming agent, to obtain the final high performance preparation.

Hereinafter, the present invention is described.
[1] A super-fatting agent,
   comprising a lipopeptide compound, squalane, an alkyl polyglycoside and a pH adjuster for neutralization or pH correction,
   wherein the lipopeptide compound is a surfactin represented by the following formula (I), an analogous compound or a salt thereof: 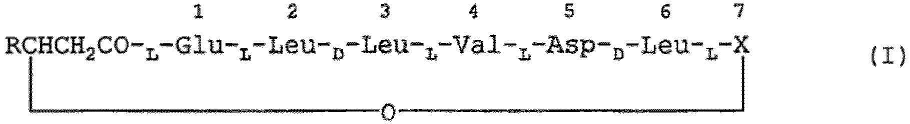
   wherein 'X' represents an amino acid residue selected from the group consisting of leucine, isoleucine, valine, glycine, serine, alanine, threonine, asparagine, glutamine, aspartic acid, glutamic acid, lysine, arginine, cysteine, methionine, phenylalanine, tyrosine, tryptophan, histidine, proline, 4-hydroxyproline and homoserine, and R is a normalalkyl group having 8 or more and 14 or less carbon atoms, an isoalkyl group having 8 or more and 14 or less carbon atoms or an anteiso-alkyl group having 8 or more and 14 or less carbon atoms, provided that the amino acids at the 2^{nd} position, the 4^{th} position and the 6^{th} position may be independently substituted by an amino acid selected from the group consisting of leucine, isoleucine, valine, glycine, serine, alanine, threonine, asparagine, glutamine, aspartic acid, glutamic acid, lysine, arginine, cysteine, methionine, phenylalanine, tyrosine, tryptophan, histidine, proline, 4-hydroxyproline and homoserine, and
   wherein a ratio of the lipopeptide compound is 0.01 mass% or more and 1 mass% or less, and a ratio of squalane is 0.5 mass% or more and 5 mass% or less, to a total of the lipopeptide compound, squalane and the alkyl polyglycoside.
[2] The super-fatting agent according to the above [1], wherein the lipopeptide compound is sodium surfactin.
[3] The super-fatting agent according to the above [1], wherein the amino acid residues at the 2^{nd} position, the 4^{th} position and the 6^{th} position are independently substituted by an amino acid selected from a group consisting of leucine, isoleucine, valine, glycine, serine, alanine, threonine, asparagine, glutamine, aspartic acid, glutamic acid, lysine, arginine, cysteine, methionine, phenylalanine, tyrosine, tryptophan, histidine, proline, 4-hydroxyproline and homoserine.
[4] The super-fatting agent according to any one of the above [1] to [3], wherein the ratio of the lipopeptide compound is 0.03 mass% or more and 0.5 mass% or less, and a ratio of squalane is 0.7 mass% or more and 3 mass% or less, to a total of the lipopeptide compound, squalane and the alkyl polyglycoside.
[5] The super-fatting agent according to any one of the above [1] to [3], wherein the ratio of the lipopeptide compound is 0.05 mass% or more and 0.1 mass% or less, and a ratio of squalane is 1 mass% or more and 2.5 mass% or less, to a total of the lipopeptide compound, squalane and the alkyl polyglycoside.
[6] A personal care formulation, comprising 0.1 mass% or more and 5 mass% or less of the super-fatting agent according to any one of the above [1] to [5].
[7] The personal care formulation according to the above [6], comprising 0.5 mass% or more and 3 mass% or less of the super-fatting agent.
[8] The personal care formulation according to the above [6] or [7], being a cosmetic.
[9] The personal care formulation according to the above [6] or [7], being a skin care cosmetic.
[10] The personal care formulation according to the above [8] or [9], being a decorative cosmetic, a face care cosmetic, a sun care cosmetic, a body care cosmetic, an antiperspirant cosmetic and a deodorant cosmetic.
[11] The personal care formulation according to the above [8] or [9], being a body wash cosmetic.
[12] The personal care formulation according to the above [8] or [9], being a cleansing cosmetic.
[13] The personal care formulation according to any one of the above [6] to [12], being in gel form or cream form.

### EFFECT OF THE INVENTION

The super-fatting agent according to the present invention, when used in a personal care formulation, stabilizes the personal care formulation, reduces skin irritating, increases skin comfort, exhibits a good wash-ability , good distribution on the skin, improves cushion property of foam, reduces foam's bubbles size, improves rinse-off effect, reduces stickiness feeling on skin, improves smoothness touch after application and long lasting.

### BRIEF DESCRIPTION OF THE DRAWINGS

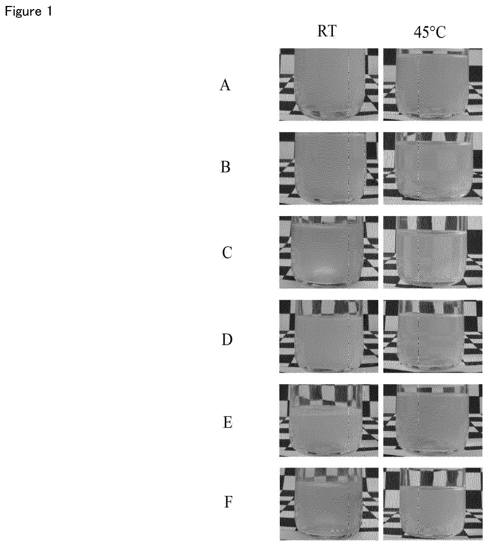

Figure 1 are photographs of the appearance of the super-fatting agent prepared in Example described later at room temperature and the appearance of the super-fatting agent incubated at 45°C for 24 hours.
Figure 2 are photographs of the appearance of the personal care formulation prepared in Example described later.
Figure 3 is a graph to demonstrate the result of a sensorial evaluation of the personal care formulation prepared in Example described later.
Figure 4 is a graph to compare the result of a sensorial evaluation of the personal care formulation prepared in Example described later with the result of a sensorial evaluation of a standard personal care base formulation.

### MODE FOR CARRYING OUT THE INVENTION

The invention is explained below in detail.

The lipopeptide compound for use in the present invention is a surfactin represented by the above formula (I), an analogous compound or a salt thereof. Examples of the lipopeptide compound used in the invention include lipopeptide compounds produced by a microorganism of genus Bacillus such as Bacillus subtilis described in JP-A-2000-327591. Preferable examples include salts of surfactin and salts of an analogous compound thereof. The lipopeptide compound is usually a compound derived from a microorganism. In the present disclosure, the "lipopeptide compound" means an interfacially active compound having a lipophilic lipid group and a water-soluble peptide part.

The surfactin herein refers to a compound represented by the formula (I) or a mixture containing two or more kinds of the compounds represented by the formula (I). For example, a mixture containing a plurality of surfactins (I) of which 'R' groups are different from each other may be used.

In the above formula (I), X represents an amino acid residue selected from the group consisting of leucine, isoleucine, valine, glycine, serine, alanine, threonine, asparagine, glutamine, aspartic acid, glutamic acid, lysine, arginine, cysteine, methionine, phenylalanine, tyrosine, tryptophan, histidine, proline, 4-hydroxyproline and homoserine. Preferred X is leucine, isoleucine or valine.

The 'R' is a normal alkyl group having 8 to 14 carbon atoms, an isoalkyl group having 8 to 14 carbon atoms or an anteiso-alkyl group having 8 to 14 carbon atoms. The normal alkyl group is a straight chain alkyl group; the isoalkyl group usually has a structure which comprises (CH₃)₂CH-(CH₂)ₙ-; and the anteiso-isoalkyl group usually has a structure which comprises CH₃-CH₂-CH(CH₃)-(CH₂)ₙ-.

The analogous compound of surfactin refers to compounds having amino acid(s) substituted by other amino acid(s) in the aforementioned formula (I), where L-leucine as the second amino acid, L-valine as the fourth amino acid and D-leucine as the sixth amino acid are each independently substituted by amino acid selected from a group consisting of leucine, isoleucine, valine, glycine, serine, alanine, threonine, asparagine, glutamine, aspartic acid, glutamic acid, lysine, arginine, cysteine, methionine, phenylalanine, tyrosine, tryptophan, histidine, proline, 4-hydroxyproline and homoserine . Hereinafter "surfactin or an analogous compound thereof" may be referred to as "surfactin".

Surfactin can be utilized as the inorganic salt or the organic salt as is seen from the above formula (I). Metal used for counter ion may be of any kind, for example, alkali metals such as sodium, potassium and lithium and alkaline earth metals such as calcium and magnesium, as long as the metal forms a salt with surfactin.

Examples of the organic salt include trimethylamine, triethylamine, tributylamine, monoethanolamine, diethanolamine, triethanolamine, lysine, arginine and choline.

Among these, sodium, potassium, monoethanolamine, diethanolamine, triethanolamine, lysine or arginine is preferred, and sodium is particularly preferred.

Sodium surfactin is on the market from KANEKA CORPORATION, under the product name of "KANEKA SUFRACTIN".

The amount of the lipopeptide compound contained in the super-fatting agent of the present invention containing the lipopeptide compound, squalane and the alkyl polyglycoside is preferably 0.01 mass% or more and 1 mass% or less, more preferably 0.03 mass% or more and 0.5 mass% or less, still more preferably 0.05 mass% or more and 0.1 mass% or less, to a total of the lipopeptide compound, squalane and the alkyl polyglycoside. When the amount is less than 0.01 mass%, the stability of the super-fatting agent may be insufficient, and when the amount is more than 1 mass%, the stability may rather deteriorate.

Squalane is a hydrocarbon and triterpene derived by hydrogenation of squalene . Squalene could be sourced from the livers of sharks or from vegetable sources like olive oil or others seed oils. Another commercial non-animal source is phyto-squalane, a compound derived from a farnesene in a sugar cane sucrose fermentation over genetically modified Saccharomyces cerevisiae yeast strains. Farnesene is dimerised to isosqualane and then hydrogenated to squalane.

The amount of squalane contained in the super-fatting agent of the present invention containing the lipopeptide compound, squalane and the alkyl polyglycoside is preferably 0.5 mass% or more and 5 mass% or less, more preferably 0.7 mass% or more and 3 mass% or less, still more preferably 1 mass% or more and 2.5 mass% or less, to a total of the lipopeptide compound, squalane and the alkyl polyglycoside. When the amount is 0.5 mass% or more, the effect by squalane, such as skin moisturizing effect and a skin protective function, may be exerted more surely, and when the amount is 5 mass%, the dispersion stability of the formulation may be ensured more certainly.

The alkyl polyglycoside in the present disclosure is a compound formed by connecting a monosaccharide or a polysaccharide and a higher alcohol through a glycosidic bond. The alkyl polyglycoside functions as a non-ionic surfactant having a lipophilic alkyl group and a water-soluble sugar part. A compound formed by connecting a monosaccharide and a higher alcohol through a glycosidic bond is idiomatically included in an alkyl polyglycoside. The alkyl polyglycoside usable in the present disclosure may be described as "an alkylmonoglycoside and/or an alkylpolyglycoside" or "at least one of an alkylmonoglycoside and an alkylpolyglycoside".

Alkyl polyglycoside (APG) used in the present invention may be used without any particular limitation, as far as the compound is conventionally employed in preparation of cosmetics. Examples of the Alkyl polyglycoside (APG) include Coco-Glucoside or Capryl-Glucoside (example Plantacare^{®} 818 UP by BASF) or Lauryl-Glucoside (example Plantacare^{®} 1200 UP by BASF) or decyl-Glucoside (example Plantacare^{®} 2000 UP by BASF) or a fatty acids derivate-Glucosides being the fatty acid a natural one going from C2 to C36 and more preferably from C4 to C24.

The amount of the alkyl polyglycoside may be appropriately adjusted, and may be the remaining part except for the lipopeptide compound and squalane in the super-fatting agent of the present invention containing the lipopeptide compound, squalane and the alkyl polyglycoside. For example, the amount of the alkyl polyglycoside is preferably 99.49 mass% or more and 94 mass% or less, more preferably 99.27 mass% or more and 96.5 mass% or less, still more preferably 98.95 mass% or more and 97.4 mass% or less, to a total of the lipopeptide compound, squalane and the alkyl polyglycoside.

The super-fatting agent according to the present invention contains a pH adjuster for neutralization or pH correction. As the pH adjuster, a general pH adjuster which is used as a component of a personal care formulation can be used. For example, an acidic pH adjuster is exemplified by a hydroxy acid such as lactic acid and glycolic acid; a dicarboxylic acid such as malic acid, tartaric acid, malonic acid, succinic acid and adipic acid; a tricarboxylic acid such as citric acid; an acidic amino acid such as glutamic acid and aspartic acid; an inorganic acid such as hydrochloric acid and phosphoric acid. A basic pH adjuster is exemplified by an alkali metal hydrogencarbonate salt such as sodium hydrogencarbonate; and an alkali metal carbonate such as sodium carbonate.

The amount of the pH adjuster in the super-fatting agent may be determined so that the pH of the super-fatting agent is appropriately adjusted. The pH of the super-fatting agent of the present invention is preferably 4.0 or more and 7.5 or less. The pH of the personal care formulation is preferably neutral or weakly acidic; however, when other component is strongly acidic, the pH of the super-fatting agent may be adjusted to 7.0 or more and 7.5 or less for neutralization. When the pH of the super-fatting agent is 4.0 or more, irritation to skin is considered to be less. The above-described pH is more preferably 7.0 or less, and even more preferably 6.5 or less, or 6.0 or less.

The personal care formulation of the present invention may contain an optional ingredient which is generally used in a general personal care formulation in addition to the super-fatting agent of the present invention in the range where the object of the invention may be achieved.

The amount of the super-fatting agent in the personal care formulation of the present invention may be appropriately adjusted, and for example, may be adjusted to 0.1 mass% or more and 5 mass% or less to the personal care formulation. When the amount is 0.1 mass% or more, the effect of the super-fatting agent of the present invention is exerted in the personal care formulation more surely. When the amount is 5 mass% or less, the effect of the other component is exerted more surely. The amount is more preferably 0.5 mass% or more and 3 mass% or less.

Examples of such an ingredient include:
hydrocarbons such as ozokerite, α-olefin oligomer, light isoparaffin, light liquid isoparaffin, squalene, squalane, synthetic squalane, phytosqualane, ceresin, paraffin, polyethylene powder, polybutene, microcrystalline wax, liquid isoparaffin, liquid paraffin, mineral oil and vaseline;
natural waxes such as jojoba oil, carnauba wax, candelilla wax, rice bran wax, shellac, lanolin, mink sebaceous wax, spermaceti wax, sugarcane wax, sperm whale oil, beeswax andmontan wax,
natural fats and fatty oils such as avocado oil, almond oil, olive oil, extra virgin olive oil, sesame seed oil, rice bran oil, rice oil, rice germ oil, corn oil, safflower oil, soybean oil, maize oil, rape seed oil, persic oil, palm kernel oil, palm oil, castor oil, sunflower oil, high oleic sunflower oil, grape seed oil, cotton seed oil, coconut oil, hydrogenated coconut oil, beef tallow, hydrogenated oil, horse oil, mink oil, yolk oil, yolk fat oil, rose hip oil, kukui nut oil, evening primrose oil, wheat germ oil, peanut oil, Camellia jeponica oil, Camellia kissi oil, cacao butter, Japan wax, beef bone tallow, nest's-foot oil, swine tallow, equine tallow, ovine tallow, shea butter, macadamia nut oil and meadowfoam seed oil;
fatty acids such as lauric acid, myristic acid, palmitic acid, stearic acid, behenic acid, oleic acid, linoleic acid, linolenic acid, γ-linolenic acid, isostearic acid, 12-hydroxystearic acid, undecylenic acid and coconut oil fatty acid;
higher alcohols such as isostearyl alcohol, octyl dodecanol, hexyl decanol, cholesterol, phytosterol, lauryl alcohol, myristyl alcohol, cetyl alcohol, stearyl alcohol, oleyl alcohol, behenyl alcohol and cetostearyl alcohol;
alkylglyceryl ethers such as batyl alcohol, chimylalcohol, serachyl alcohol and isostearyl glyceryl ether;
esters such as isopropyl myristate, butyl myristate, isopropylpalmitate, ethyl stearate, butyl stearate, ethyl oleate, ethyl linoleate, isopropyl linoleate, cetyl caprylate, hexyl laurate, isooctylmyristate, decylmyristate, myristylmyristate, cetyl myristate, octadecyl myristate, cetyl palmitate, stearyl stearate, decyl oleate, oleyl oleate, cetyl ricinoleate, isostearyl laurate, isotridecyl myristate, isocetyl myristate, isostearyl myristate, octyldodecyl myristate, 2-ethylhexyl palmitate, isocetyl palmitate, isostearyl palmitate, 2-ethylhexyl stearate, isocetyl stearate, isodecyl oleate, octyldodecyl oleate, octyldodecyl ricinoleate, ethyl isostearate, isopropyl isostearate, cetyl 2-ethylhexanoate, cetostearyl 2-ethylhexanoate, stearyl 2-ethylhexanoate, hexyl isostearate, ethylene glycol dioctanoate, ethylene glycol dioleate, propylene glycol dicaprylate, propylene glycol dicaprylate/dicaprate, propylene glycol dicaprate, propylene glycol dioleate, neopentyl glycol dicaprate, neopentyl glycol dioctanoate, glyceryl tricaprylate, glyceryl tri 2-ethyl hexanoate, glyceryl tricaprylate/tricaprate, glyceryl tricaprylate/tricaprate/tristearate, glyceryl triundecylate, glyceryl triisopalmitate, glyceryl triisostearate, trimethylolpropane tri 2-ethylhexanoate, trimethylolpropane triisostearate, pentaerythrityl tetra 2-ethylhexanoate, pentaerythrityl tetramyristate, pentaerythrityl tetraisostearate, diglyceryl tetraisostearate, octyldodecyl neopentanotae, isocetyl octanoate, isostearyl octanoate, 2-ethylhexyl isopelargonate, hexyldecyl dimethyloctanoate, octyldodecyl dimethyloctanoate, 2-ethylhexyl isopalmitate, isocetyl isostearate, isostearyl isostearate, octyldodecyl isostearate, lauryl lactate, myristyl lactate, cetyl lactate, octyldodecyllactate, triethyl citrate, acetyltriethyl citrate, acetyltributyl citrate, trioctyl citrate, triisocetyl citrate, trioctyldodecyl citrate, diisostearyl malate, 2-ethylhexyl hydroxystearate, di 2-ethylhexyl succinate, diisopropyl adipate, diisobutyl adipate, dioctyl adipate, diheptylundecyl adipate, sebacate diethyl, diisopropyl sebacate, dioctyl sebacate, cholesteryl stearate, cholesteryl isostearate, cholesteryl hydroxystearate, cholesteryloleate, dihydrocholesteryl oleate, phytosteryl isostearate, phytosteryl oleate, isocetyl 12-stearoyl hydroxystearate, stearyl 12-stearoyl hydroxystearate, isostearyl 12-stearoyl hydroxystearate, polyoxyethylene (3) polyoxypropylene (1) cetyl ether acetate, polyoxyethylene (3) polyoxypropylene (1) isocetyl ether acetate, isononyl isononanoate, octyl isononanoate, tridecyl isononanoate and isotridecyl isononanoate;
silicone oils such as methyl polysiloxane, methylphenyl polysiloxane, methyl hydrogen polysiloxane, methyl cyclopolysiloxane, octamethyl cyclotetrasiloxane, decamethyl cyclopentasiloxane, dodecamethyl cyclohexasiloxane, octamethyl trisiloxane, decamethyl tetrasiloxane, tetradecamethyl hexasiloxane, highly polymerized methyl polysiloxane, dimethylsiloxane-methyl (polyoxyethylene) siloxane-methyl (poly oxypropylene)siloxane copolymer, dimethylsiloxane-methyl(polyoxyethylene)siloxane copolymer, dimethylsiloxane-methyl(polyoxypropylene)siloxane copolymer, dimethylsiloxane-methylcetyl oxysiloxane copolymer, dimethylsiloxane-methyl stearoxysiloxane copolymer, polyether modified silicone, alcohol modified silicone, alkyl modified silicone and amino modified silicone;
polyhydric alcohols such as ethylene glycol, diethylene glycol, triethylene glycol, polyethylene glycol, propylene glycol, dipropylene glycol, polypropylene glycol, glycerin, diglycerin, polyglycerin, 3-methyl-1,3-butanediol, 1,3-butanediol, 1,2-pentanediol and 1,2-hexanediol;
saccharides such as mannitol, sorbitol, xylitol, maltitol, erythritol, pentaerythritol, glucose, sucrose, fructose, lactose, maltose, xylose and trehalose;
polymers such as sodium alginate, carrageen, agar, furcellaran, guar gum, quince seed, Amorphophalus konjak (arum root) mannan, tamarind gum, tara gum, dextrin, starch, locust bean gum, gum arabic, gum gatti, karaya gum, gum tragacanth, arabinogalactan, pectin, quince, chitosan, starch, curdlan, xanthan gum, gellan gum, cyclodextrin, dextran, pullulan, microcrystalline cellulose, methyl cellulose, ethyl cellulose, hydroxyethyl cellulose, hydroxypropyl cellulose, hydroxypropylmethyl cellulose, carboxymethyl cellulose, carboxy starch, cationized cellulose, starch phosphate ester, cationized guar gum, carboxymethyl-hydroxypropylated guar gum, hydroxypropylated guar gum, albumin, casein, gelatin, sodium polyacrylate, polyacrylic amide, carboxyvinyl polymer, polyethylene imine, highly polymerized polyethylene glycol, polyvinyl alcohol, polyvinyl pyrrolidone, polyvinyl ether, polyacryl amide, acrylic acid copolymer, methacrylic acid copolymer, maleic acid copolymer, vinylpyridine copolymer, ethylene/acrylic acid copolymer, vinyl pyrrolidone based polymer, vinylalcohol/vinyl pyrrolidone copolymer, nitrogen-substituted acrylamide based polymer, amino modified silicone, cationized polymer, dimethylacryl ammoniumbasedpolymer, acrylic acidbased anion polymer, methacrylic acid based anion polymer, modified silicone, acrylate/methacrylate alkyl (C 10 to 30) copolymer and polyoxyethylene/polyoxypropylene copolymer;
alcohols such as ethanol, isopropyl alcohol, 1-butanol, 2-butanol and benzyl alcohol;
anionic surfactants such as coconut oil fatty acid potassium, coconut oil fatty acid sodium, coconut oil fatty acid triethanolamine, potassium laurate, sodium laurate, triethanolamine laurate, potassium myristate, sodium myristate, isopropanolamine myristate, potassium palmitate, sodium palmitate, isopropanolamine palmitate, potassium stearate, sodium stearate, triethanolamine stearate, potassium oleate, sodium oleate, castor oil fatty acid sodium, zinc undecylate, zinc laurate, zinc myristate, magnesium myristate, zincpalmitate, zinc stearate, calcium stearate, magnesium stearate, aluminum stearate, calcium myristate, magnesium myristate, aluminum dimyristate, aluminum isostearate, polyoxyethylene lauryl ether acetate, sodium polyoxyethylene lauryl ether acetate, polyoxyethylene tridecyl ether acetate, sodium polyoxyethylene tridecyl ether acetate, sodium stearoyl lactate, sodium isostearoyl lactate, sodium lauroyl sarcosine, coconut oil fatty acid sarcosine, sodium coconut oil fatty acid sarcosine, coconut oil fatty acid sarcosine triethanolamine, lauroyl sarcosine, potassium lauroyl sarcosine, lauroyl sarcosine triethanolamine, oleoyl sarcosine, sodium myristoyl sarcosine, sodium stearoyl glutamate, coconut oil fatty acid acyl glutamic acid, potassium coconut oil fatty acid acyl glutamate, sodium coconut oil fatty acid acyl glutamate, coconut oil fatty acid acyl glutamate triethanolamine, lauroylacyl glutamic acid, potassium lauroylacyl glutamate, sodium lauroylacyl glutamate, lauroylacyl glutamate triethanolamine, myristoylacyl glutamic acid, potassium myristoylacyl glutamate, sodium myristoylacyl glutamate, stearoylacyl glutamic acid, potassium stearoylacyl glutamate, disodium stearoylacyl glutamate, sodium hydrogenated beef tallow fatty acid acyl glutamate, sodium coconut oil fatty acid/hydrogenated beef tallow fatty acid acyl glutamate, sodium coconut oil fatty acid methylalanine, lauroyl methylalanine, sodium lauroyl methylalanine, lauroyl methylalanine triethanolamine, sodium myristoyl methylalanine, sodium lauroyl methyltaurine, potassium coconut oil fatty acid methyltaurine, sodium coconutoil fatty acid methyltaurine, magnesium coconut oil fatty acid methyltaurine, sodium myristoyl methyltaurine, sodium palmitoyl methyltaurine, sodium stearoyl methyltaurine, sodium oleoyl methyltaurine, sodium alkane sulfonate, sodium tetradecene sulfonate, sodium sulfosuccinate dioctyl, disodium lauryl sulfosuccinate, sodium coconut oil fatty acid ethyl ester sulfonate, sodium lauryl sulfate, triethanolamine lauryl sulfate, sodium cetyl sulfate, triethanolamine alkyl (11, 13, 15) sulfate, sodium alkyl (12, 13) sulfate, triethanolamine alkyl (12, 13) sulfate, alkyl (12, 14, 16) ammonium sulfate, diethanolamine alkyl (12 to 13) sulfate, triethanolamine alkyl (12 to 14) sulfate, triethanolamine alkyl (12 to 15) sulfate, magnesium coconut oil alkyl sulfate/triethanolamine, lauryl ammonium sulfate, potassium lauryl sulfate, magnesiumlauryl sulfate, monoethanolamine lauryl sulfate, diethanolamine lauryl sulfate, sodium myristyl sulfate, sodium stearylsulfate, sodium oleyl sulfate, triethanolamine oleyl sulfate, sodium polyoxyethylene lauryl ether sulfate, triethanolaminepolyoxyethylene lauryl ether sulfate, sodium polyoxyethylene (1) alkyl (11, 13, 15) ether sulfate, triethanolamine polyoxyethylene (1) alkyl (11, 13, 15) ether sulfate, sodium polyoxyethylene (3) alkyl (11 to 15) ether sulfate, sodiumpolyoxyethylene (2) alkyl (12, 13) ether sulfate, sodium polyoxyethylene (3) alkyl (12 to 14) ether sulfate, sodium polyoxyethylene (3) alkyl (12 to 15) ether sulfate, sodium polyoxyethylene (2) lauryl ether sulfate, sodiumpolyoxyethylene (3) myristyl ether sulfate, sodium higher fatty acid alkanol amide sulfate ester, lauryl phosphate, sodium lauryl phosphate, potassium cetyl phosphate, diethanolamine cetyl phosphate, polyoxyethylene oleyl ether phosphate, polyoxyethylene lauryl ether phosphate, sodium polyoxyethylene lauryl ether phosphate, polyoxyethylene cetyl ether phosphate, sodium polyoxyethylene cetyl ether phosphate, polyoxyethylene stearyl ether phosphate, polyoxyethylene oleyl ether phosphate, sodium polyoxyethylene oleyl ether phosphate, polyoxyethylene alkylphenyl ether phosphate, sodium polyoxyethylene alkylphenyl ether phosphate, triethanolamine polyoxyethylene alkylphenyl ether phosphate, polyoxyethylene octyl ether phosphate, polyoxyethylene (10) alkyl (12, 13) ether phosphate, polyoxyethylene alkyl (12 to 15) etherphosphate, polyoxyethylene alkyl (12 to 16) etherphosphate, triethanolamine polyoxyethylene lauryl ether phosphate and diethanolamine polyoxyethylene oleyl ether phosphate;
cationic surfactants such as dioctylamine, dimethylstearylamine, trilaurylamine, diethylaminoethylamide stearate, lauryl trimethylammonium chloride, cetyl trimethylammonium chloride, cetyl trimethylammonium bromide, cetyl trimethylammonium saccharin, stearyl trimethylammonium chloride, alkyl (20 to 22) trimethylammonium chloride, lauryl trimethylammonium bromide, alkyl (16, 18) trimethylammonium chloride, stearyl trimethylammonium bromide, stearyl trimethylammonium saccharin, alkyl (28) trimethylammonium chloride, di(polyoxyethylene) oleyl methylammonium (2EO) chloride, dipolyoxyethylene stearyl methylammonium chloride, polyoxyethylene (1) polyoxypropylene (25) diethylmethylammonium chloride, tri (polyoxyethylene) stearyl ammonium (5EO) chloride, distearyl dimethylammonium chloride, dialkyl (12 to 15) dimethylammonium chloride, dialkyl (12 to 18) dimethylammonium chloride, dialkyl (14 to 18) dimethylammonium chloride, dicocoyl dimethylammonium chloride, dicetyl dimethylammonium chloride, isostearyllauryl dimethylammonium chloride, benzalkonium chloride, myristyl dimethylbenzyl ammonium chloride, lauryl dimethyl (ethylbenzyl) ammonium chloride, stearyl dimethylbenzyl ammonium chloride, lauryl pyridinium chloride, cetyl pyridinium chloride, lauroyl cholamino formylmethyl pyridinium chloride, stearoyl cholamino formylmethyl pyridinium chloride, alkyl isoquinolinium bromide, methyl benzethonium chloride and benzethonium chloride;
ampholytic surfactants such as 2-alkyl-N-carboxymethyl-N-hydroxyethyl imidazolium betaine, alkyldiamino ethyl glycine hydrochloride, sodium lauryldiamino ethyl glycine, sodium undecyl hydroxyethyl imidazolium betaine, undecyl-N-carboxymethyl imidazolium betaine, disodium coconut oil fatty acid acyl-N-carboxyethyl-N-hydroxyethyl ethylenediamine, disodium coconut oil fatty acid acyl-N-carboxyethoxyethyl-N-carboxyethyl ethylenediamine, disodium coconut oil fatty acid acyl-N-carboxymethoxyethyl-N-carboxymethyl ethylenediamine, sodium laurylamino propionate, sodium laurylamino dipropionate, triethanolamine laurylamino propionate, sodium palm oil fatty acid acyl-N-carboxyethyl-N-hydroxyethyl ethylenediamine, betaine lauryldimethylamino acetate, betaine coconut oil alkyldimethylamino acetate, betaine stearyl dimethylamino acetate, sodium stearyldimethyl betaine, coconut oil fatty acid amidopropyl betaine, palm oil fatty acid amidopropyl betaine, amidopropyl acetate betaine laurate, amidopropyl betaine ricinoleate, stearyl dihydroxyethyl betaine and lauryl hydroxysulfobetaine;
nonionic surfactants such as polyoxyethylene (10) alkyl (12, 13) ether, polyoxyethylene lauryl ether, polyoxyethylene cetyl ether, polyoxyethylene stearyl ether, polyoxyethylene oleyl ether, polyoxyethylene (3, 7, 12) alkyl (12 to 14) ether, polyoxyethylene tridecyl ether, polyoxyethylene myristyl ether, polyoxyethylene-sec-alkyl (14) ether, polyoxyethylene isocetyl ether, polyoxyethylene cetostearyl ether, polyoxyethylene (2, 10, 20) isostearyl ether, polyoxyethylene oleylcetyl ether, polyoxyethylene (20) arachyl ether, polyoxyethylene octyldodecyl ether, polyoxyethylene behenyl ether, polyoxyethylene octylphenyl ether, polyoxyethylene nonylphenyl ether, polyoxyethylene dinonylphenyl ether, polyoxyethylene (1) polyoxypropylene (1, 2, 4, 8) cetyl ether, polyoxyethylene (5) polyoxypropylene (1, 2, 4, 8) cetyl ether, polyoxyethylene (10) polyoxypropylene (1, 2, 4, 8) cetyl ether, polyoxyethylene (20) polyoxypropylene (1, 2, 4, 8) cetyl ether, polyoxyethylene polyoxypropylene lauryl ether, polyoxyethylene (3) polyoxypropylene (34) stearyl ether, polyoxyethylene (4) polyoxypropylene (30) stearyl ether, polyoxyethylene (34) polyoxypropylene (23) stearyl ether, polyoxyethylene polyoxypropylene cetyl ether, polyoxyethylene polyoxypropylene decyltetradecyl ether, polyethylene glycol monolaurate, ethylene glycol monostearate, polyethylene glycol monostearate, polyethylene glycolmonooleate, ethylene glycol fatty acid ester, self-emulsifying ethylene glycol monostearate, diethylene glycol laurate, polyethylene glycol myristate, polyethylene glycol palmitate, diethylene glycol stearate, self-emulsifying polyethylene glycol (2) monostearate, polyethylene glycol isostearate, ethylene glycol dioctanoate, diethylene glycol dilaurate, polyethylene glycol dilaurate, polyethylene glycol (150) dipalmitate, ethylene glycol distearate, diethylene glycol distearate, polyethylene glycol distearate, ethylene glycol dioleate, polyethylene glycol dioleate, polyethylene glycol diricinoleate, polyoxyethylene (20) sorbitan monolaurate, polyoxyethylene (20) sorbitan monopalmitate, polyoxyethylene (6) sorbitan monostearate, polyoxyethylene (20) sorbitan monostearate, polyoxyethylene (20) sorbitan tristearate, polyoxyethylene (6) sorbitan monooleate, polyoxyethylene (20) sorbitan monooleate, polyoxyethylene (20) sorbitan trioleate, polyoxyethylene (20) coconut oil fatty acid sorbitan, polyoxyethylene (10 to 80) sorbitanmonolaurate, polyoxyethylene sorbitan tristearate, polyoxyethylene (20) sorbitan isostearate, polyoxyethylene (150) sorbitan tristearate, polyoxyethylene castor oil, polyoxyethylene hydrogenated castor oil, polyoxyethylene (10) hydrogenated castor oil, polyoxyethylene (20) hydrogenated castor oil, polyoxyethylene (40) hydrogenated castor oil, polyoxyethylene (50) hydrogenated castor oil, polyoxyethylene (60) hydrogenated castor oil, lipophilic glycerin monostearate, lipophilic glycerin monooleate, self-emulsifying glycerin monostearate, coconut oil fatty acid glyceryl, glycerin laurate, glyceryl myristate, glyceryl isostearate, glyceryl ricinoleate, glyceryl monohydroxystearate, glycerin oleate, glyceryl linoleate, glyceryl erucate, glyceryl behenate, wheat germoil fatty acid glyceride, safflower oil fatty acid glyceryl, hydrogenated soybean fatty acid glyceryl, saturated fatty acid glyceride, cotton seed oil fatty acid glyceryl, monomyristate glyceryl monoisostearate, mono tallowate glyceride, monolanolin fatty acid glyceryl, glyceryl sesquioleate, glyceryl distearate, glyceryl diisostearate, glyceryl diarachidate, sorbitan monolaurate, sorbitan monopalmitate, sorbitan monostearate, sorbitan monoisostearate, sorbitan monooleate, sorbitan sesquistearate, sorbitan sesquioleate, sorbitan tristearate, sorbitan trioleate, coconut oil fatty acid sorbitan, sorbitan isostearate, sorbitan sesquiisostearate, sorbitan distearate, diglyceryl isopalmitate, poly(4 to 10)glyceryl monolaurate, poly(10)glyceryl monomyristate, poly(2 to 10)glyceryl monostearate, poly(2 to 10) glyceryl monoisostearate, poly (2 to 10) glyceryl monooleate, diglyceryl sesquioleate, poly(2 to 10)glyceryl diisostearate, poly(6 to 10)glyceryl distearate, diglyceryl triisostearate, poly(10)glyceryl tristearate, poly(10)glyceryl trioleate, poly(2)glyceryl tetraisostearate, decaglyceryl pentastearate, poly(6 to 10)glyceryl pentaoleate, poly(10)glyceryl heptastearate, decaglyceryl decastearate, poly(10)glyceryl decaoleate, concentrated poly (6) glyceryl ricinoleate, sucrose fatty acid ester, coconut oil fatty acid sucrose ester, alkyl glucoside, coconut oil alkyl dimethylamine oxide, lauryl dimethylamine oxide, dihydroxyethyl lauryl dimethylamine oxide, stearyl dimethylamine oxide, oleyl dimethylamine oxide and polyoxyethylene coconut oil alkyl dimethylamine oxide;
natural surfactants such as saponin, lecithin, soybean phospholipid, hydrogenated soybean phospholipid, soybean lysophospholipid, hydrogenated soybean lysophospholipid, yolk lecithin, hydrogenated yolk lysophosphatidylcholine, phosphatidylcholine, phosphatidylethanolamine, phosphatidylserine, sphingophospholipid, sphingomyelin, ganglioside, bile acid, cholic acid, deoxycholic acid, sodium cholate, sodium deoxycholate, spiculisporic acid, rhamnolipid, trehalose lipid, sophorolipid and mannosyl erythritol lipid;
ultraviolet ray absorbers such as: para-aminobenzoic acid derivatives such as para-aminobenzoic acid, ethyl paraaminobenzoate, glyceryl para-aminobenzoate, amyl para-dimethyl aminobenzoate and 2-ethylhexyl para-dimethyl aminobenzoate; cinnamic acid derivatives such as benzyl cinnamate, mono-2-ethyl hexanoate glyceryl dipara-methoxycinnamate, methyl 2,4-diisopropyl cinnamate, ethyl 2,4-diisopropyl cinnamate, potassium para-methoxycinnamate, sodium para-methoxycinnamate, isopropyl para-methoxycinnamate, 2-ethylhexyl para-methoxycinnamate, 2-ethoxyethyl paramethoxycinnamate and ethyl para-ethoxycinnamate; urocanic acid derivatives such as urocanic acid and ethyl urocanate; benzophenone derivatives such as 2,4-dihydroxybenzophenone, 2,2',4,4'-tetrahydroxybenzophenone, sodium 2-hydroxy-4-methoxy-5-sulfobenzophenone, 2-hydroxy-4-methoxybenzophenone-5-sulfonate, 2-hydroxy-4-methoxybenzophenone, 2,2'-dihydroxy-4,4'-dimethoxybenzophenone and sodium 2,2'-dihydroxy-4,4'-dimethoxy-5-sulfobenzophenone; salicylic acid derivatives such as ethylene glycol salicylate, salicylate-2-ethylhexyl, phenyl salicylate, benzyl salicylate, p-tert-butylphenyl salicylate, homomenthyl salicylate and salicylate-3,3,5-trimethylcyclohexyl; 2-(2'-hydroxy-5'-methoxyphenyl)benzotriazole and 4-tert-butyl-4'-methoxybenzoyl methane;
powders and color materials such as: kaolin, silicic anhydride, magnesium aluminum silicate, sericite, talc, boron nitride, mica, montmorillonite, hemp cellulose powder, wheat starch, silk powder, maize starch; natural dyes such as nitro dyes, azo dyes, nitroso dyes, triphenylmethane dyes, xanthene dyes, quinoline dyes, anthraquinone dyes, indigo dyes, pyrene dyes, phthalocyanine dyes, flavonoid, quinone, porphyrin, water soluble annatto, sepia powder, caramel, guaiazulene, gardenia blue, gardenia yellow, cochineal, shikonin, sodium copper chlorophyllin, paprika dye, safflower red, safflower yellow, laccaic acid and riboflavin butyrate ester; carbon black, yellow iron oxide, black iron oxide, red iron oxide, iron blue, ultramarine blue, zinc oxide, chromium oxide, titanium oxide, black titanium oxide, zirconium oxide, chromium hydroxide, alumina, magnesium oxide, barium sulfate, aluminum hydroxide, calcium carbonate, lithium cobalt titanate, manganese violet and pearl pigment,
plant extracts such as Angelica keiskei extract, Uncaria gambir extract, avocado extract, sweet hydrangea leaf extract, Gynostemmapentaphyllum makino extract, Althaea officinalis extract, Arnica montana extract, oil soluble Arnica montana extract, almond extract, aloe extract, Japanese styrax benzoin extract, Ginkgo biloba extract, Stinging nettle extract, Orris rhizome root extract, fennel extract, turmeric extract, dog rose fruit extract, Echinacea leaf extract, Scutellaria root extract, Phellodendron bark extract, Japanese captis extract, barley extract, okura extract, Hypericum perforatum extract, oil soluble Hypericum perforatum extract, Lamium album extract, oil soluble Lamium album extract, Ononis spinosa root extract, Nasturtium of ficinale extract, orange extract, orange flower water, seaweed extract, persimmon tannin, pueraria root extract, Japanese valerian extract, cattail extract, Chamomile(matricaria) extract, oil soluble Chamomile (matricaria) extract, Chamomile (matricaria) distillate, Avena sativa (oat) kernel extract, carrot extract, oil soluble carrot extract, carrot oil, Artemisia capillaris extract, Glycyrrhiza glabra (licorice) extract, powdered Glycyrrhiza glabra (licorice) extract, Glycyrrhiza glabra (licorice) extract flavonoid, cantharides tincture, raspberry extract, kiwi extract, cinchona extract, cucum ber extract, apricot kernel extract, quince seed extract, gardenia florida extract, Sasaalbomarginata extract, Sophoraroot extract, walnut shell extract, Citrus paradisi (grapefruit) extract, Clematis vitalba leaf extract, black sugar extract, chlorella extract, mulberry bark extract, Cinnamon bark extract, Gentian extract, Geranium herb extract, black tea extract, Nuphar extract, burdock root extract, oil soluble burdock root extract, wheat germ extract, hydrolyzed wheat powder, rice bran extract, fermented rice bran extract, Symphytum officinale (comfrey) extract, Asiasarum root extract, Crocus sativus (saffron) extract, Saponaria officinalis extract, oil soluble salvia extract, Crataegus cuneata fruit extract, Zanthoxylum fruit extract, Lentinus edodes extract, powdered Lentinus edodes extract, Rehmannia root extract, Lithospermum root extract, oil soluble Lithospermum root extract, Perilla herb extract, linden extract, oil soluble Tilia europaea extract, Filipendula extract, Peony root extract, Coix lacryma-jobi extract, ginger extract, oil soluble ginger extract, ginger tincture, Acorus calamus root extract, Betula pendula (birch) extract, oil soluble Betula alba (birch) extract, Betula pendula (birch) sap, Lonicera japonica extract, Equisetum arvense extract, oil soluble Equisetum arvense extract, scordinin, stevia extract, ivy extract, Crataegus oxyacantaha (whitethorn) extract, sambucus extract, Juniperus communis extract, Achillea milefolium extract, oil soluble Achillea milefolium extract, Mentha piperita (peppermint) extract, Salvia officinalis (sage) extract, oil soluble Salvia officinalis (sage) extract, Salvia officinalis (sage) water, Malva Sylvestris (mallow) extract, Apium graveolens (celery) extract, Cnidium officinale extract, Cnidium officinale water, Swertia herb extract, Glycine max (soybean) extract, Jujube extract, thyme extract, green tea extract, tea leaf dry distilled solution, tea seed extract, clove extract, Citrus unshiu peel extract, Camellia japonica extract, Centella asiatica extract, oil soluble walnut extract, duku extract, Terminalia sericea extract, Capsicum tincture, Japanese angelica root extract, oil soluble Japanese angelica root extract, Japanese angelica root water, Calendula officinalis flower extract, oil soluble Calendula officinalis flower extract, soy milk powder, peach seed extract, Bitter orange peel extract, Houttuynia cordata extract, Solanum lycopersicum (tomato) extract, Potentilla tormentilla Schrk (Rosaceae) extract, fermented soybeans extract, Ginseng extract, oil soluble Ginseng extract, Allium sativum (garlic) extract, wild rose extract, oil soluble wild rose extract, malt extract, malt root extract, Ophiopogon tuber extract, parsley extract, rye leaf juice concentrate, peppermint distillate, witch hazel distillate, witch hazel extract, rose extract, parietaria extract, Isodonis japonicus extract, Eriobotrya japonica leaf extract, oil soluble Eriobotrya japonica leaf extract, coltsfoot extract, hoelen extract, Ruscus aculeatus root extract, powdered Ruscus aculeatus root extract, grape extract, grape leaf extract, grape water, Hayflower extract, Luffa cylindrica fruit extract, Luffa cylindrica fruit water, Carthamus tinctorius (safflower) extract, oil soluble Tilia platyphyllos extract, linden distillate, Paeonia suffruticosa (peony) extract, Humulus lupulus (hops) extract, oilsoluble Humuluslupulus (hops) extract, pine extract, Silybum marianum (milk thistle) extract, Aesculus hippocastanum (horse chestnut) extract, oil soluble Aesculus hippocastanum (horse chestnut) extract, Sapindus mukurossi extract, Melissa officinalis (balm mint) extract, Melilotus officinalis (melilot) extract, Prunus persica(peach)leaf extract, oil soluble Prunus persica (peach) leaf extract, bean sprouts extract, Centaurea cyanus flower extract, Centaurea cyanus flower distillate, Eucalyptus globulus extract, Saxifrage extract, Lilium (lily) extract, Coix seed extract, oil soluble Coix seed extract, Artemisia princeps pampanini extract, Artemisia princeps pampanini water, Lavandula angustifolia (lavender) extract, Lavandula angustifolia (lavender) water, apple extract, Ganoderma lucidum extract, Lactuca sativa (lettuce) extract, lemon extract, Astragalus sinicus extract, Rosa centifolia (rose) flower water, Rosemarinus officinalis (rosemary) extract, oil soluble Rosemarinus officinalis (rosemary) extract, Anthemis nobilis extract and Sanguisorba officinalis extract;
amino acids and peptides such as glycine, alanine, valine, leucine, isoleucine, serine, threonine, phenylalanine, tyrosine, tryptophan, cystine, cysteine, methionine, proline, hydroxyproline, aspartic acid, asparagine, glutamic acid, glutamine, arginine, histidine, lysine, γ-aminobutyric acid, DL-pyrrolidonecarboxylic acid, ε-aminocaproic acid, hydrolyzed elastin, water soluble elastin, hydrolyzed collagen, water soluble collagen, casein, glutathione, wheat peptides and soybean peptide;
vitamins and factors acting like a vitamin such as: vitamin A and analogues thereof such as retinol, retinal, retinoic acid, retinol acetate and retinol palmitate; carotenoids such as α-carotene, β-carotene, γ-carotene, δ-carotene, lycopene, zeaxanthin, cryptoxanthin, echinenon and astaxanthin; vitamin B1 and analogues thereof such as thiamines; vitamin B2 and analogues thereof such as riboflavin; vitamin B6 and analogues thereof such as pyridoxine, pyridoxal and pyridoxamine; vitamin B12 and analogues thereof such as cyanocobalamin; folic acids, nicotinic acid, nicotinamide, pantothenic acids, biotins; vitamin C and analogues thereof such as L-ascorbic acid, sodium L-ascorbate, L-ascorbyl stearate, Lascorbyl palmitate, L-ascorbyl dipalmitate, L-ascorbyl tetraisopalmitate, L-ascorbate sulfate disodium ester, magnesium L-ascorbyl, sodium L-ascorbyl phosphate and L-ascorbate-2-glucoside; vitamin D and analogues thereof such as ergocalciferol and cholecalciferol; vitamin E and analogues thereof such as d-α-tocopherol, DL-α-tocopherol, dl-α-tocopherol acetate, dl-α-tocopherol succinate, β-tocopherol, γ-tocopherol and d-δ-tocopherol; ubiquinones, vitaminKand analogues thereof, carnitine, ferulic acid, γ-oryzanol, α-lipoic acid and orotic acid;
antiseptic agents such as benzoic acid, sodium benzoate, undecylenic acid, salicylic acid, sorbic acid, potassium sorbate, dehydroacetic acid, sodium dehydroacetate, isobutyl paraoxybenzoate, isopropyl paraoxybenzoate, ethyl paraoxybenzoate, butyl paraoxybenzoate, propyl paraoxybenzoate, benzyl paraoxybenzoate, methyl paraoxybenzoate, sodium paraoxybenzoate methyl, phenoxyethanol, light sensitive dye No. 101, light sensitive dye No. 201 and light sensitive dye No. 401;
antioxidizing agents such as butylhydroxyanisole, butylhydroxytoluene, propyl gallate, erythorbic acid, sodium erythorbate, para-hydroxyanisole and octyl gallate;
chelating agents to bind to a metal ion such as trisodium ethylenediamine hydroxyethyl triacetate, edetic acid, disodium edetate, trisodium edetate, tetrasodium edetate, sodium citrate, gluconic acid, phytic acid, sodium polyphosphate and sodium metaphosphate;
moisturizing agents such as hyaluronic acid, sodium hyaluronate, sodium chondroitin sulfate, sodium lactate, sodium pyrrolidone carboxylate, betaine, lactic acidbacteria fermented solution, yeast extract and ceramide;
anti-inflammatory agents such as glycyrrhizic acid, trisodium glycyrrhizinate, dipotassium glycyrrhizinate, monoammonium glycyrrhizinate, β-glycyrrhetinic acid, glycerin glycyrrhetinate, stearyl glycyrrhetinate, lysozyme chloride, hydrocortisone and allantoin;
salts such as sodium chloride, potassium chloride, magnesium chloride and sodium sulfate;
α-hydroxy acids such as citric acid, glycolic acid, tartaric acid and lactic acid;
whitening agents such as arbutin, α-arbutin and placenta extract;
essential oils such as Archangelica officinalis (angelica) oil, Canangium odoratum (ylang ylang) oil, Canarium luzonicum (elemi) oil, orange oil, Chamomilla recutita (matricaria) oil, Anthemis nobilis oil, Elettaria cardamom (cardamon) oil, Acorus calamus (calamus) oil, Ferula galbaniflua (galbanum) oil, Cinnamomum camphora (camphor) oil, Daucus carota (carrot) seed oil, Salviasclarea (clarysage) oil, Citrusparadisi (grapefruit) oil, Eugenia caryophyllus (clove) oil, Cinnamon bark oil, Coriandrum sativum (coriander) oil, Cupressus sempervirens (cypress) oil, Santalum album (sandalwood) oil, Juniperus virginiana (cedarwood) oil, Cympogon nardus (citronella) oil, Cinnamomum zeylanicum (Cinnamon) leaf oil, Jasmine officinale (jasmine) absolute oil, Juniperus communis (juniper Berry) oil, Zingiber officinale (ginger) extract, Menthaspicata (spearmint) oil, Salvia officinalis (sage) oil, cedar oil, Pelargonium grabeolens (geranium) oil, Thymus vulgaris (thyme) oil, Melaleuca alternifolia (tea tree) oil, Myristica fragrans (nutmeg) oil, Melaleuca qui.viridiflara (niaouli) oil, Citrus aurantium (neroli) oil, pine oil, Ocimum basilicum (basil) oil, Mentha arvensis oil, Pogostemon patchouli (patchouli) oil, Cymbopogon martini (palmarosa) oil, Foeniculumvulgare (fennel) oil, Citrus bigaradia (petitgrain) oil, Piper nigrum (black pepper) oil, Boswellia carterii (frankincense) oil, Vetiveria zizanoides (vetivert) oil, Menthapiperita (peppermint) oil, Citrusbergamia (bergamot) oil, benzoin oil, Aniba rosaeodora (bois de rose) oil, Origanum majorana (marjoram) oil, mandarin oil, Conumiphora myrrha (myrrh) oil, Melissa officinalis (balm mint) oil, Eucalyptus globulus oil, Citrus junos oil, Citrus aurantifolia (lime) oil, Ravensare aromaticum (ravensare) oil, Lavandula latifolia (lavandin) oil, Lavandula angustifolia (lavender) oil, Tilia vulgaris (linden) oil, lemon oil, lemon grass oil, rose oil, Aniba rosaeodora (rosewood) oil, Rosemarinus officinalis (rosemary) oil and Levisticum officinale (lovage) oil;
terpenes such as limonene, pinene, terpinene, terpinolene, myrcene and longifeelene;
fragrance, and the like.

Furthermore, to the cosmetic among the personal care formulation of the present invention may also be added any existing raw material of cosmetics at a general concentration. All raw materials of cosmetics described in, for example, Keshouhin genryou kizyun dai-2han chukai (Glossary for Standards of Cosmetic Ingredients, 2nd edition) edited by Society of Japanese Pharmacopoeia, 1984 (YAKUJI NIPPO LIMITED.); Keshouhin genryou kizyun-gai seibun kikaku (The Japanese Cosmetic Ingredients Codex), under the editorship of Pharmaceutical Affairs Bureau Evaluation and Registration Division, 1993 (YAKUJI NIPPO LIMITED.), Keshouhin genryou kizyun-gai seibun kikaku tsuiho (Supplement to The Japanese Cosmetic Ingredients Codex), under the editorship of Pharmaceutical Affairs Bureau Evaluation and Registration Division, 1993 (YAKUJI NIPPO LIMITED.), Keshouhin syubetsu kyoka kizyun (The Comprehensive Licensing Standards of Cosmetics by Category) , under the editorship of Pharmaceutical Affairs Bureau Evaluation and Registration Division, 1993 (YAKUJI NIPPOLIMITED. ) , Keshouhin syubetsu haigou seibun kikaku (The Japanese Cosmetic IngredientsCodexby Category), under the editorship of Pharmaceutical Affairs Bureau Evaluation and Registration Division, 1997 (YAKUJI NIPPO LIMITED.), Keshouhin genryou jiten (Dictionary of rawmaterials of cosmetics), 1991 (Nikko Chemicals Co., Ltd.) and the like may be used.

The amount of the other component except for the super-fatting agent in the personal care formulation of the present invention is preferably 0.01 mass% or more and 80 mass% or less, more preferably 0.1 mass% or more and 25 mass% or less, and still more preferably 0.3 mass% or more and 10 mass% or less in the total amount of the personal care formulation.

The super-fatting agent of the present invention obtained in the method as above causes no irritation on skin, and is extremely excellent as: basic skin care cosmetics such as skin toner, lotion, milky lotion, essence, skin cream, cleansing gel, cleansing cream and pack; makeup cosmetics such as makeup base, foundation, eye shadow, lip color and lip gloss; massaging cosmetics such as massaging gel and massaging cream (cold cream); and cosmetics for hair such as hair cream, styling gel and hair wax.

When a personal care formulation containing a surfactant is contacted with a skin and hair, a problem such as dry skin, dry hair and skin roughness may be caused in some cases by excessively rinsing away a lipophilic component such as sebum. Accordingly, a super-fatting agent containing a lipophilic component may be contained in a personal care formulation. There is however possibility that the stability of a personal care formulation may be possibly impaired by adding a super-fatting agent, since a main solvent of a personal care formulation is water. On the one hand, the super-fatting agent of the present invention can remarkably improve the dispersibility of a lipophilic component in a personal care formulation containing water as a main solvent, since the super-fatting agent of the present invention contains a specific amount of a lipopeptide compound an alkyl polyglycoside as surfactants in addition to squalane as a lipophilic component. In addition, the super-fatting agent of the present invention has low irritativeness to the human skin. Furthermore, the super-fatting agent of the present invention, when incorporated in a composition, can improve a texture, washing performance, rinsing performance and moisture-retaining property. The super-fatting agent of the present invention is therefore is very useful as a component of a personal care formulation, particularly one which is contacted with skin. The personal care formulation is exemplified by a cosmetic, such as a skin care cosmetic, a decorative cosmetic, a face care cosmetic, a sun care cosmetic, a body care cosmetic, an antiperspirant cosmetic, a deodorant cosmetic and a cleansing cosmetic. As a cleaning agent, a body wash cosmetic is exemplified.

The dosage form of the super-fatting agent of the present invention is not particular restricted and may be appropriately selected, and is exemplified by gel and cream. The dosage form of the personal care formulation of the present invention is exemplified by skin toner, lotion, milky lotion, essence, cream, pack, makeup base, makeup cosmetics, massaging cosmetics and cleansing cosmetics. Among them, a cleansing cosmetic is suitable because the property upon washing away with water becomes favorable .

The super-fatting agent and personal care formulation of the present invention can be produced according to a conventional method. More specifically, the super-fatting agent and personal care formulation can be prepared using a generally utilized stirring apparatus or a generally utilized emulsification apparatus. Moreover, in instances where a cleansing cosmetic in gel form is produced, it can be also prepared by such a method as one disclosed in JP-A-2003-176211.

### EXAMPLES

The present invention is explained in more detail below by way of Examples, however the invention is by no means limited to these Examples.

In the following Examples, "KANEKA SUFRACTIN" manufactured by KANEKA CORPORATION was used as sodium surfactin, "%" of each component represents a percentage by mass.

Example 1: Production of formulation samples
1) The pH of coco-glucoside ("818 Plantacare (registered mark)" manufactured by BASF) was adjusted to 4.5 - 5.5 by adding 50% citric acid aqueous solution. As a result, the coco-glucoside solution became transparent. The coco-glucoside was C₈₋₁₆ fatty alcohol glucoside.
2) Into the cold coco-glucoside solution with stirring at 200 rpm using a paddle, sodium surfaction was added in a ratio described in Table 1.
3) After sodium surfactin was completely dissolved, squalane was added in a ratio described in Table 1 to be homogeneously dispersed into the cooled and stirred solution. The amount of C₈₋₁₆ fatty alcohol glucoside in the formulation samples A to F was adjusted so that the total amount of sodium surfactin, squalane and C₈₋₁₆ fatty alcohol glucoside was 100%.

**Table 1**

| Sample No. | Squalane | Sodium Surfactin | C₈₋₁₆ Fatty alcohol glucoside |
|---|---|---|---|
| A | 2% | 1.0 - 3.0% | remnant |
| B | 2% | 0.1 - 1.0% | remnant |
| C | 2% | 0.05 - 0.1% | remnant |
| D | 2% | 0.03 - 0.05% | remnant |
| E | 2% | 0.01 - 0.03% | remnant |
| F | 2% | 0.005 - 0.01% | remnant |

### Test example 1: Evaluation of visual appearance

The visual appearance of the each formulation samples was observed at 45°C or room temperature.

All samples A to F turned out to be turbid at roor temperature. In particular, the samples A and F were very turbid. On the one hand, the visual appearance of the samples B to E were improved after 24 hours at 45°C. In particular, the visual appearance of the formulation sample C containing 0.05 to 0.1% of sodium surfactin was remarkably improved as the formulation sample C was slightly turbid in yellow but almost transparent.

In addition, the formulation sample C was incubated at 45°C for 24 hours, and then additionally stood still for 1 week at room temperature, but the sample did not regress to the initial turbidity and almost transparent appearance was maintained.

As the above-described result, the formulation sample C was the most preferred as a super-fatting agent. The choice was dictated by the fact that the formulation sample C was the one with the best performance of stability over time and the best transparency to 45°C and room temperature. The super-fatting agent in the following tests was the formulation sample C.

### Test example 2: Analysis of viscosity

The viscosity, density and solid content of the formulation sample C were measured. The result is shown as follows.
- Viscosity (viscometer: Brookfield RVDV-E, measurement temperature: 20°C, spindle: 2, rotational speed: 60 rpm) - 428.7 mPas
- Density - 1.095 g/mL
- Solid content (thermobalance was used, 105°C, AUTO) - 55.6%
- Solid content (thermobalance was used, 105°C, 2h) - 53.0%

Into a personal care formulation having the composition described in Table 2, the formulation sample C was added as a super-fatting agent at 1% or 2%. As a control to be compared, formulation sample Z which was a mixture of C₈₋₁₆ fatty alcohol glucoside and glyceryl oleate was prepared and similarly added into the basic personal care formulation.

**Table 2**

| Product name | INCI name | Ratio |
|---|---|---|
| Sabosol EMS | sodium laureth sulfate | 35% |
| EuTraSoft 675 SB | cocamidopropylhydroxysultaine | 5% |
| Demi water | water | remnant |
| NaCl | sodium chloride | 2% |
| Lactid acid 80% | lactic acid aqueous solution | pH 4.5-5.5 |
| Microcare PM2 | phenoxyethanol | 0.7% |
| | ethylparaben | |
| | methylparaben | |
| | propylene glycol | |

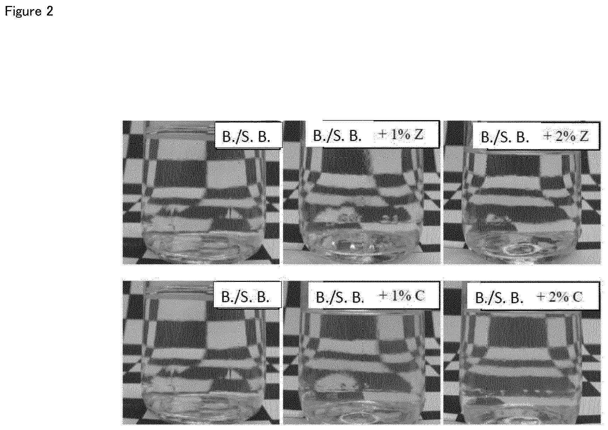

The photographs of visual appearance of the above-described basic personal care formulation itself and the personal care formulation which contained the formulation sample C or formulation sample Z are shown as Figure 2. In Figure 2, the basic personal care formulation is described as "B./S.B.", which represents Bath/Shower Base.

As Figure 2, the personal care formulation containing the formulation sample C or formulation sample Z was transparent. In the personal care formulation which contained 2% of the formulation sample C, a misty thin layer was formed; but the whole visual appearance was highly transparent.

In addition, the viscosity of each personal care formulation was measured. As a viscometer, "Brookfield RVDV-E" manufactured by Brookfield AMETEK was used. As a spindle, RV-3 was used. The measurement temperature was adjusted to 23°C, and the rotational speed was adjusted to 2.5 rpm.

**Table 3**

| | Concentration of sample C or Z | Viscosity | |
|---|---|---|---|
| B/S. B. | - | 24,880 mPas | control |
| B/S. B. + sample C | 1% | 20,000 mPas | viscosity was decreased in comparison with control |
| | 2% | 11,680 mPas | |
| B/S. **B.** + **sample** Z | 1% | 35,040 mPas | viscosity was increased in comparison with control |
| | 2% | 45,100 mPas | |

The viscosity of the Bath/Shower Base was clearly reduced by the formulation sample C. On the one hand, when the formulation sample Z was added, the viscosity was increased. When the concentration of the formulation sample was increased up to 2%, the viscosity of the personal care formulation was further increased in the case of the formulation sample Z but the viscosity was further reduced in the case of the formulation sample C.

### Test example 3: Evaluation of volume and structure of foam personal care formulation

Into a 500 mL graduated cylinder with ground neck and cap, a 0.5% aqueous solution of each formulation described in Table 4. After the cylinder was vigorously shaken 15 times, the volume, size and persistence of foams were evaluated. The result is shown in Table 4.

**Table 4**

| | Volume of large foam | Volume of small foam | Persistence of foam |
|---|---|---|---|
| B/S. B. | 270 mL | 190 mL | good |
| B/S. B. + 1% sample C | 280 mL (good) | 200 mL | good |
| B/S. B. + 2% sample C | 280 mL | 240 mL (good) | good |
| B/S. B. + 1% sample Z | 270 mL | 200 mL | good |
| B/S. B. + 2% sample Z | 280 mL | 200 mL | good |

Even though the formulation sample C or the formulation sample Z was added to the basic formulation, the volume of foams was not decreased and the persistence of foams was not affected. The properties were slightly improved by the addition of the formulation sample C, but there was not large difference of the volume and size of foams between the cases of the addition of the formulation sample C and the formulation sample Z. The structure of small foams of the formulation which contained the formulation sample C or the formulation sample Z was denser than that of the basic formulation.

### Test example 4: Sensorial evaluation

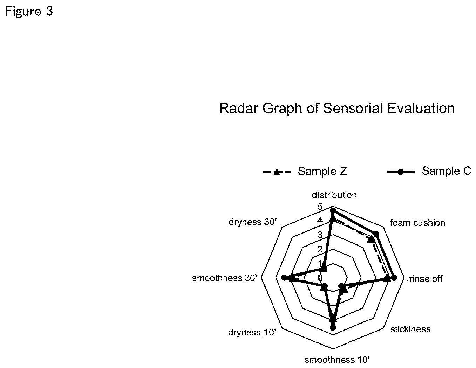

On the palm, 1 g of each formulation was added and 1 g of tap water was added. The oil and fat component inside of the antebrachial region was preliminarily removed without using a cleaning agent, and the inside was washed using the formulation for 15 seconds. After the inside was rinsed off, the inside was tapped. The subject evaluated the following items on a five level scale in which "1" was good and "5" was bad. The result is shown in Table 5 and Figure 3.

**Table 5**

| Item | Sample Z | Sample C | Difference (C-Z) |
|---|---|---|---|
| distribution | 4.2 | 4.7 | 0.5 |
| foam cushion | 3.8 | 4.3 | 0.5 |
| rinse off | 3.8 | 4.3 | 0.5 |
| stickiness | 1.1 | 0.83 | -0.27 |
| smoothness 10' | 2.8 | 3.5 | 0.7 |
| dryness 10' | 0.92 | 0.83 | -0.09 |
| smoothness 30' | 2.8 | 3.4 | 0.6 |
| dryness 30' | 1.0 | 0.92 | -0.08 |

With respect to distribution (application feeling), foam cushion, rinse off and smoothness after 10 minutes and 30 minutes, the Bath/Shower Base containing the formulation sample C was superior to the Bath/Shower Base containing the formulation sample Z. On the one hand, with stickiness and dryness after 10 minutes and 30 minutes, the formulation containing the formulation sample C was relatively lower and containing the formulation sample Z was relatively higher.

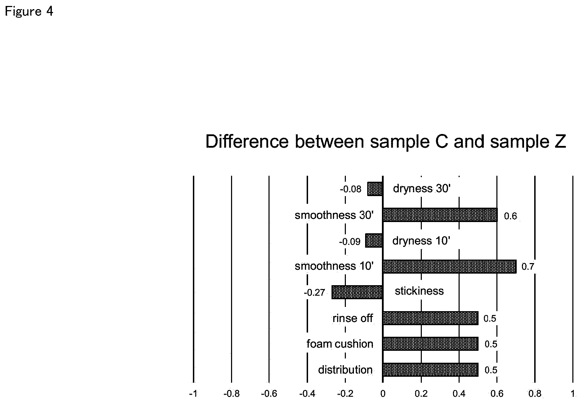

In order to clearly demonstrate that the formulation sample C containing sodium surfactin is superior to the formulation sample Z, which is a mixture of C₈₋₁₆ fatty alcohol glucoside and glyceryl oleate, the result of the formulation containing the formulation sample Z is converted to zero to prepare a graph. The graph is shown as Figure 4. A positive or negative average of each evaluated item of the formulation containing the formulation sample C is emphasized in Figure 4, and Figure 4 demonstrated excellent skin distribution, softness of the foam, rinse off, and less dryness and stickiness.

### Test example 5: Evaluation of performance stability

Even after the formulation sample C was incubated at room temperature, 45°C in an oven or 5°C in a refrigerator for a long time, the formulation was stable. In addition, the transparency of the formulation in an oven and at room temperature after 2 months was further improved. All of the personal care formulations were stable after 2 months in the above-described each condition.

### CONCLUSIONS

- All the formulation samples A, B, C, D, E and F became turbid conditions in room temperature. The formulation samples B, C, D and E were improved at 45°C after 24 hours, and particularly the formulation sample C was very transparent.
- Furthermore, even when the formulation sample C heated at 45°C was stood still at room temperature for 1 week, the formulation sample C amazingly did not become turbid again and the visual appearance remained unchanged.
- All personal care formulations containing the formulation sample C or formulation sample Z were transparent.
- The appearances of the personal care formulations containing 1% or 2% of the formulation sample Z or formulation sample C were compared with the Bath/Shower Base formulation without the components. The personal care formulation containing 2% of the formulation sample C had a thin layer of haze, but the high transparency thereof was confirmed.
- The viscosity of the Bath/Shower formulation to which the formulation sample C was added was reduced in comparison with the Bath/Shower Base without the formulation sample C. On the one hand, when the formulation sample Z was added, the viscosity was increased. In addition, when the concentration of the formulation sample C was increased to 2%, the viscosity of the personal care formulation according to the present invention was increased in comparison with the Bath/Shower formulation containing 2% of the formulation sample Z.
- The foam of the Bath/Shower formulation containing the formulation sample C or formulation sample Z was not decreased due to re-fatting and maintained as an advantageous characteristic for a Bath/Shower Base formulation in all of 3 examples. When the volume and structure of foam of the Bath/Shower Base formulations containing the formulation sample C and formulation sample Z were compared, a larger amount of a smaller foam was produced by the formulation containing 2% of the formulation sample C in comparison with the case of the formulation containing the formulation sample C.
- Between the Bath/Shower formulations containing the formulation sample C and formulation sample Z in the same concentration, different effect on a sensorial level was shown. For example, the formulation containing the formulation sample C exhibited higher distribution property, could be applied uniformly on the skin and was more easily rinsed off in comparison with the formulation containing the formulation sample Z. The foam of the formulation containing squalane was softer than that of the formulation containing glyceryl oleate, and was excellent in the filming effect and softness on the skin and stabilization property. In addition, the Bath/Shower formulation containing the formulation sample Z was slightly sticky immediately after drying; but the Bath/Shower formulation containing the formulation sample C exhibited a little and elasticity.

### INDUSTRIAL APPLICABILITY

The personal care formulation containing the super-fatting agent according to the present invention has an extremely low skin irritating property, ensures high skin comfort and exhibits unexpected storage stability.

## Claims

1. A super-fatting agent,
comprising a lipopeptide compound, squalane, an alkyl polyglycoside and a pH adjuster for neutralization or pH correction,
wherein the lipopeptide compound is a surfactin represented by the following formula (I), an analogous compound or a salt thereof: 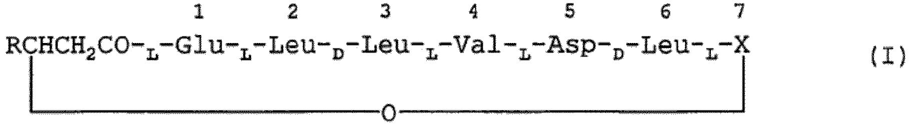 wherein 'X' represents an amino acid residue selected from the group consisting of leucine, isoleucine, valine, glycine, serine, alanine, threonine, asparagine, glutamine, aspartic acid, glutamic acid, lysine, arginine, cysteine, methionine, phenylalanine, tyrosine, tryptophan, histidine, proline, 4-hydroxyproline and homoserine, and R is a normalalkyl group having 8 or more and 14 or less carbon atoms, an isoalkyl group having 8 or more and 14 or less carbon atoms or an anteiso-alkyl group having 8 or more and 14 or less carbon atoms, provided that the amino acids at the 2^{nd} position, the 4^{th} position and the 6^{th} position may be independently substituted by an amino acid selected from the group consisting of leucine, isoleucine, valine, glycine, serine, alanine, threonine, asparagine, glutamine, aspartic acid, glutamic acid, lysine, arginine, cysteine, methionine, phenylalanine, tyrosine, tryptophan, histidine, proline, 4-hydroxyproline and homoserine, and
wherein a ratio of the lipopeptide compound is 0.01 mass% or more and 1 mass% or less, and a ratio of squalane is 0.5 mass% or more and 5 mass% or less, to a total of the lipopeptide compound, squalane and the alkyl polyglycoside.

2. The super-fatting agent according to claim 1, wherein the lipopeptide compound is sodium surfactin.

3. The super-fatting agent according to claim 1, wherein the amino acid residues at the 2^{nd} position, the 4^{th} position and the 6^{th} position are independently substituted by an amino acid selected from a group consisting of leucine, isoleucine, valine, glycine, serine, alanine, threonine, asparagine, glutamine, aspartic acid, glutamic acid, lysine, arginine, cysteine, methionine, phenylalanine, tyrosine, tryptophan, histidine, proline, 4-hydroxyproline and homoserine.

4. The super-fatting agent according to any one of claims 1 to 3, wherein the ratio of the lipopeptide compound is 0.03 mass% or more and 0.5 mass% or less, and a ratio of squalane is 0.7 mass% or more and 3 mass% or less, to a total of the lipopeptide compound, squalane and the alkyl polyglycoside.

5. The super-fatting agent according to any one of claims 1 to 3, wherein the ratio of the lipopeptide compound is 0.05 mass% or more and 0.1 mass% or less, and a ratio of squalane is 1 mass% or more and 2.5 mass% or less, to a total of the lipopeptide compound, squalane and the alkyl polyglycoside.

6. A personal care formulation, comprising 0.1 mass% or more and 5 mass% or less of the super-fatting agent according to any one of claims 1 to 5.

7. The personal care formulation according to claim 6, comprising 0.5 mass% or more and 3 mass% or less of the super-fatting agent.

8. The personal care formulation according to claim 6 or 7, being a cosmetic.

9. The personal care formulation according to claim 6 or 7, being a skin care cosmetic.

10. The personal care formulation according to claim 8 or 9, being a decorative cosmetic, a face care cosmetic, a sun care cosmetic, a body care cosmetic, an antiperspirant cosmetic or a deodorant cosmetic.

11. The personal care formulation according to claim 8 or 9, being a body wash cosmetic.

12. The personal care formulation according to claim 8 or 9, being a cleansing cosmetic.

13. The personal care formulation according to any one of claims 6 to 12, being in gel form or cream form.

## Patentansprüche

1. Überfettungsmittel,
umfassend eine Lipopeptidverbindung, Squalan, ein AlkylPolyglykosid und ein pH-Einstellmittel zur Neutralisierung oder pH-Korrektur,
wobei die Lipopeptidverbindung ein Surfactin der folgenden Formel (I), eine analoge Verbindung oder ein Salz davon, ist: 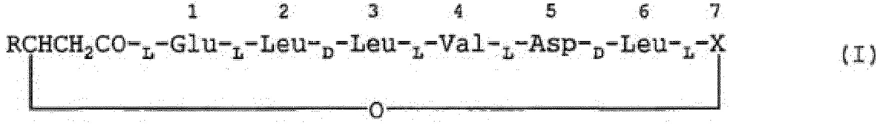
worin 'X' einen Aminosäurerest darstellt, ausgewählt aus der Gruppe, bestehend aus Leucin, Isoleucin, Valin, Glycin, Serin, Alanin, Threonin, Asparagin, Glutamin, Asparaginsäure, Glutaminsäure, Lysin, Arginin, Cystin, Methionin, Phenylalanin, Tyrosin, Tryptophan, Histidin, Prolin, 4-Hydroxyprolin und Homoserin, und R eine Normalalkylgruppe mit 8 oder mehr und 14 oder weniger Kohlenstoffatomen, eine Isoalkylgruppe mit 8 oder mehr und 14 oder weniger Kohlenstoffatomen oder eine Anteisoalkylgruppe mit 8 oder mehr und 14 oder weniger Kohlenstoffatomen, ist, mit der Maßgabe, dass die Aminosäuren an der 2. Position, der 4. Position und der 6. Position unabhängig voneinander durch eine Aminosäure substituiert sein können, ausgewählt aus der Gruppe, bestehend aus Leucin, Isoleucin, Valin, Glycin, Serin, Alanin, Threonin, Asparagin, Glutamin, Asparaginsäure, Glutaminsäure, Lysin, Arginin, Cystin, Methionin, Phenylalanin, Tyrosin, Tryptophan, Histidin, Prolin, 4-Hydroxyprolin und Homoserin, und
wobei ein Verhältnis der Lipopeptidverbindung 0,01 Masse-% oder mehr und 1 Masse-% oder weniger beträgt, und ein Verhältnis von Squalan 0,5 Masse-% oder mehr und 5 Masse-% oder weniger beträgt, bezogen auf die Gesamtmenge der Lipopeptidverbindung, des Squalans und des Alkyl-Polyglykosids.

2. Überfettungsmittel gemäß Anspruch 1, wobei die Lipopeptidverbindung Natriumsurfactin ist.

3. Überfettungsmittel gemäß Anspruch 1, wobei die Aminosäurereste an der 2. Position, der 4. Position und der 6. Position, unabhängig voneinander durch eine Aminosäure substituiert sind, ausgewählt aus der Gruppe, bestehend aus Leucin, Isoleucin, Valin, Glycin, Serin, Alanin, Threonin, Asparagin, Glutamin, Asparaginsäure, Glutaminsäure, Lysin, Arginin, Cystin, Methionin, Phenylalanin, Tyrosin, Tryptophan, Histidin, Prolin, 4-Hydroxyprolin und Homoserin.

4. Überfettungsmittel gemäß mindestens einem der Ansprüche 1 bis 3, wobei das Verhältnis der Lipopeptidverbindung 0,03 Masse-% oder mehr und 0,5 Masse-% oder weniger beträgt, und ein Verhältnis von Squalan 0,7 Masse-% oder mehr und 3 Masse-% oder weniger beträgt, bezogen auf die Gesamtmenge der Lipopeptidverbindung, des Squalans und des Alkyl-Polyglykosids.

5. Überfettungsmittel gemäß mindestens einem der Ansprüche 1 bis 3, wobei das Verhältnis der Lipopeptidverbindung 0,05 Masse-% oder mehr und 0,1 Masse-% oder weniger beträgt, und ein Verhältnis von Squalan 1 Masse-% oder mehr und 2,5 Masse-% oder weniger beträgt, bezogen auf die Gesamtmenge der Lipopeptidverbindung, des Squalans und des Alkyl-Polyglykosids.

6. Körperpflegezusammensetzung, umfassend 0,1 Masse-% oder mehr und 5 Masse-% oder weniger des Überfettungsmittels gemäß mindestens einem der Ansprüche 1 bis 5.

7. Körperpflegezusammensetzung gemäß Anspruch 6, umfassend 0,5 Masse-% oder mehr und 3 Masse-% oder weniger des Überfettungsmittels.

8. Körperpflegezusammensetzung gemäß Anspruch 6 oder 7, die ein Kosmetikum ist.

9. Körperpflegezusammensetzung gemäß Anspruch 6 oder 7, die ein Hautpflegekosmetikum ist.

10. Körperpflegezusammensetzung gemäß Anspruch 8 oder 9, die ein dekoratives Kosmetikum, ein Gesichtspflegekosmetikum, ein Sonnenpflegekosmetikum, ein Körperpflegekosmetikum, ein Antitranspirant-Kosmetikum oder ein Deodorant-Kosmetikum, ist.

11. Körperpflegezusammensetzung gemäß Anspruch 8 oder 9, die ein Körperwaschkosmetikum ist.

12. Körperpflegezusammensetzung gemäß Anspruch 8 oder 9, die ein reinigendes Kosmetikum ist.

13. Körperpflegezusammensetzung gemäß mindestens einem der Ansprüche 6 bis 12, die in Gel- oder Cremeform ist.

## Revendications

1. Agent surgraissant,
comprenant un composé lipopeptidique, un squalène, un polyglycoside d'alkyle et un régulateur de pH pour la neutralisation ou la correction du pH,
dans lequel le composé lipopeptidique est une surfactine représentée par la formule (I) suivante, un composé analogue ou un sel de celui-ci : 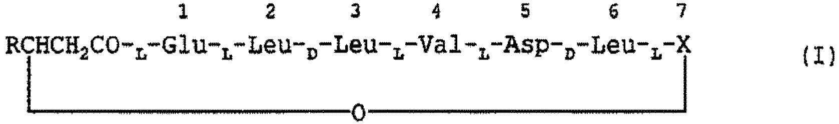
dans lequel 'X' représente un résidu d'acide aminé choisi parmi le groupe consistant en leucine, isoleucine, valine, glycine, sérine, alanine, thréonine, asparagine, glutamine, acide aspartique, acide glutamique, lysine, arginine, cystéine, méthionine, phénylalanine, tyrosine, tryptophane, histidine, proline, 4-hydroxyproline et homosérine, et R représente un groupe alkyle normal présentant 8 atomes de carbones ou plus et 14 atomes de carbone ou moins, un groupe isoalkyle présentant 8 atomes de carbone ou plus et 14 atomes de carbone ou moins ou un groupe antéiso-alkyle présentant 8 atomes de carbone ou plus et 14 atomes de carbone ou moins, à condition que les acides aminés en 2^{ème} position, en 4^{ème} position et en 6^{ème} position puissent être substitués indépendamment par un acide aminé choisi parmi le groupe consistant en leucine, isoleucine, valine, glycine, sérine, alanine, thréonine, asparagine, glutamine, acide aspartique, acide glutamique, lysine, arginine, cystéine, méthionine, phénylalanine, tyrosine, tryptophane, histidine, proline, 4-hydroxyproline et homosérine, et
dans lequel un rapport du composé lipopeptidique est supérieur ou égal à 0,01 % en masse et inférieur ou égal à 1 % en masse, et un rapport de squalène est supérieur ou égal à 0,5 % en masse et inférieur ou égal à 5 % en masse, sur un total du composé lipopeptidique, du squalène et du polyglycoside d'alkyle.

2. Agent surgraissant selon la revendication 1, dans lequel le composé lipopeptidique est une surfactine sodique.

3. Agent surgraissant selon la revendication 1, dans lequel les résidus d'acides aminés en 2^{ème} position, en 4^{ème} position et en 6^{ème} position sont substitués indépendamment par un acide aminé choisi parmi le groupe consistant en leucine, isoleucine, valine, glycine, sérine, alanine, thréonine, asparagine, glutamine, acide aspartique, acide glutamique, lysine, arginine, cystéine, méthionine, phénylalanine, tyrosine, tryptophane, histidine, proline, 4-hydroxyproline et homosérine.

4. Agent surgraissant selon l'une quelconque des revendications 1 à 3, dans lequel le rapport du composé lipopeptidique est supérieur ou égal à 0,03 % en masse et inférieur ou égal à 0,5 % en masse, et un rapport de squalène est supérieur ou égal à 0,7 % en masse et inférieur ou égal à 3 % en masse, sur un total du composé lipopeptidique, du squalène et du polyglycoside d'alkyle.

5. Agent surgraissant selon l'une quelconque des revendications 1 à 3, dans lequel le rapport du composé lipopeptidique est supérieur ou égal à 0,05 % en masse et inférieur ou égal à 0,1 % en masse, et un rapport de squalène est supérieur ou égal à 1 % en masse et inférieur ou égal à 2,5 % en masse, sur un total du composé lipopeptidique, du squalène et du polyglycoside d'alkyle.

6. Formulation de soins d'hygiène personnelle, comprenant 0,1 % en masse ou plus et 5 % en masse ou moins de l'agent surgraissant selon l'une quelconque des revendications 1 à 5.

7. Formulation de soins d'hygiène personnelle selon la revendication 6, comprenant 0,5 % en masse ou plus et 3 % en masse ou moins de l'agent surgraissant.

8. Formulation de soins d'hygiène personnelle selon la revendication 6 ou 7, étant un produit cosmétique.

9. Formulation de soins d'hygiène personnelle selon la revendications 6 ou 7, étant un produit cosmétique de soins pour la peau.

10. Formulation de soins d'hygiène personnelle selon la revendication 8 ou 9, étant un produit cosmétique de maquillage, un produit cosmétique de soins du visage, un produit cosmétique de protection solaire, un produit cosmétique de soins du corps, un produit cosmétique de type antitranspirant ou un produit cosmétique de type déodorant.

11. Formulation de soins d'hygiène personnelle selon la revendication 8 ou 9, étant un produit cosmétique de nettoyant pour le corps.

12. Formulation de soins d'hygiène personnelle selon la revendication 8 ou 9, étant un produit cosmétique de nettoyage.

13. Formulation de soins d'hygiène personnelle selon l'une quelconque des revendications 6 à 12, étant sous forme de gel ou sous forme de crème.
